Europäisches Patentamt

European Patent Office

Office européen des brevets

(11) Veröffentlichungsnummer: **0 548 595 A2**

# (12) EUROPÄISCHE PATENTANMELDUNG

(21) Anmeldenummer: **92120434.3**

(22) Anmeldetag: **30.11.92**

(51) Int. Cl.5: **A61K 9/48**, A61K 31/44

(30) Priorität: **12.12.91 IT MI913335**

(43) Veröffentlichungstag der Anmeldung:
**30.06.93 Patentblatt 93/26**

(84) Benannte Vertragsstaaten:
**AT BE CH DE DK ES FR GB GR IE IT LI LU MC NL PT SE**

(71) Anmelder: **Bayer S.p.A.**
**Viale Certosa 126**
**I-20156 Milano(IT)**

(72) Erfinder: **Ciceri, Silvana**
**Via Galilei 1**
**Como, Milano(IT)**
Erfinder: **Dondi, Gilberto**
**Via Dei Tigli 13**
**Cusano Milanino, Milano(IT)**
Erfinder: **Scurati, Paolo**
**Via Card, Riboldi 5**
**Paderno Dugnano, Milano(IT)**

(74) Vertreter: **Müller, Gerhard, Dr. et al**
**BAYER AG Konzernverwaltung RP**
**Patentabteilung**
**W-5090 Leverkusen 1 Bayerwerk (DE)**

(54) **Pharmazeutisches System zur Verabreichung von therapeutisch wirksamen Dihydropyridinen mit gesteuerter Freisetzung, und Herstellungsverfahren.**

(57) Die Erfindung betrifft ein pharmazeutisches System zur Verabreichung von therapeutisch wirksamen Dihydropyridinen mit gesteuerter Freisetzung. Das genannte pharmazeutische System besteht aus einer Hartgelatinkapsel, die eine Kombination von drei oder mehr Tabletten mit unterschiedlich eingestellter Wirkstoff-Freisetzung enthält. Auch die Herstellung eines solchen pharmazeutischen Systems wird beschrieben.

EP 0 548 595 A2

Die vorliegende Erfindung betrifft ein pharmazeutisches System zur Verabreichung von therapeutisch wirksamen Dihydropyridinen mit gesteuerter Freisetzung, sowie ein Verfahren zu dessen Herstellung.

Die pharmakologische Behandlung einer Krankheit besteht bekanntlich aus der sofortigen und kontinuierlichen Verabreichung eines Arzneimittels.

Im allgemeinen umfaßt die Behandlung eine erste rasch zu resorbierende Dosis und darauffolgende Dosen, die zur Aufrechterhaltung von therapeutisch wirksamen Konzentrationen des Arzneimittels im Blutplasma bestimmt sind.

Die Wirksamkeit der Behandlung hängt seinerseits von der zuverlässigen Mitwirkung des Patienten bei der Aufnahme der Arzneimittel dem durch den behandelnden Arzt festgelegten therapeutischen Plan gemäß ab.

Obwohl es unverständlich erscheinen kann, selbst wenn es sich um ein wesentliches Wohl des Menschen, wie die Gesundheit, handelt, sind Unterlassung und Vergessen bei der Aufnahme der Arzneimittel gar nicht selten.

Dieses international als "patient non compliance" genannte Verhalten ist wohlbekannt und wurde zahlreich auch vom psychologischen Gesichtspunkt untersucht.

Die Notwendigkeit, dieses Problem an seiner Wurzel zu lösen, hat zur Entwicklung von pharmazeutischen Formen geführt, die gattungsgemäß als Formen mit anhaltender Wirkung bezeichnet werden können, womit die tägliche Aufnahme zahlenmäßig vermindert werden konnte. Verschiedene Systeme mit anhaltender Arzneimittelfreisetzung sind bekannt bzw. wurden experimentell untersucht. Grundsätzlich sind zwei Mechanismen möglich: dem einen liegt die gesteuerte Abgabe des Arzneimittels, dem anderen eine wiederholte Abgabe zugrunde. Unter den ersten seien diejenigen Systeme genannt, die auf Diffusionsprozessen durch durchlässige Überzüge bzw. Filme basieren, diejenigen, die auf hydrophobe bzw. hydrophyle Matrizen und diejenigen Systeme, die auf Ionenaustausch basieren, usw.

Die Systeme der zweiten Gruppe bestehen üblicherweise aus Einzeldosen, die mit Überzügen bzw. Filmen verschiedener Beschaffenheit beschichtet, bei verschiedenen pH-Werten löslich sind.

Aestetisch-mathematische Überlegungen haben viele berühmte Autoren dazu gebracht, die erste Lösung a priori zu bevorzugen; jedoch ist die vorliegende Anmelderin der Meinung, daß kein rationeller Zugang zur Lösung dieser Aufgabe ohne pharmakokinetischen Überlegungen möglich ist.

Insbesondere im Falle von Wirkstoffen, die einen erhöhten "first pass effect" (d.h. der stoffwechselmäßige Effekt des ersten Leberdurchganges) aufweisen, kann eine pulsierende Verabreichung den Vorteil haben, die Überschreitung der wirksamen Mindestkonzentration des Arzneimittels im Blut sicherzustellen bzw. besonderen klinischen Anforderungen nachzukommen.

Dem Fachmann ist bekannt, daß die Dihydropyridinen, deren wohlbekannteste Stammverbindung Niphendipin ist, dieser Arzneimittelklasse gehören. Im allgemeinen sind diese Stoffe ausreichend wirksam, damit sie für jegliche Formulierungsweise, einschliesslich die sogenannte pulsierende Abgabe, geeignet sind. Vielmehr, ist dieses letztgenannte System eine jedenfalls wertvolle Lösung und könte sogar die bevorzugte Methode bei sehr schwer löslichen Dihydropyridinen oder bei Dihydropyridinen, die irgendwo im Darmtrakt mikrobiologisch abgebaut werden können sein, darstellen.

Beispielsweise ist die Reduzierung der Anzahl der Verabreichungen mit Nimodipin - ein Wirkstoff, der zu dieser letzten Klasse gehört - deswegen besonders vorteilhaft, da eine seiner wichtigsten Indikationen die Behandlung von kronischen ischaemischen Gehirnsyndromen bei älteren Patienten ist.

Bekanntlich sieht im allgemeinen die gegenwärtig angewendete Therapie die Verabreichung dreier Tabletten pro Tag vor: es bestand daher der Bedarf die tägliche Anzahl der Verabreichungen auf zwei, vorzugsweise auf eine einzuschränken.

Es wurde nun gefunden, daß solches Ziel dadurch erreicht werden kann, indem man das pharmazeutische System zur Verabreichung mit gesteuerter Freisetzung der vorliegenden Erfindung verwendet, wie es in den Patentansprüchen definiert ist.

Die Erfindung wird im folgenden anhand Anwendungsbeispiele näher erläutert. Nachdem der Fachmann von den Prinzipien der Herstellung des pharmazeutischen Systems zur Verabreichung von therapeutisch wirksamen Dihydropyridinen mit gesteuerter Freisetzung der vorliegenden Erfindung gemäß Kenntnis genommen hat, wird er ohne jegliche Schwierigkeit die Verfahrensparameter den besonderen Bedürfnissen anpassen können, ohne vom Erfindungsgedanken abzuweichen.

Die tägliche Behandlung der beschriebenen Methode gemäß kann je nach den Eigenschaften des Wirkstoffes auch aus der Verabreichung einer einzigen Dosis pro Tag bestehen. Die Arzneimittelabgabe kann durch eine geeignete Kombination der verschiedenen Filme bzw. Filmdicke gesteuert werden.

**Beispiel 1**

**Herstellung eines pharmazeutischen Systems zur Verabreichung von Nimodipin mit gesteuerter Freisetzung.**

Es werden 164,8 mg-Tabletten hergestellt, die 15 mg therapeutisch wirksame Substanz pro Tablette enthalten anhand folgender Einsatzstoffe:

| Nimodipin | 5 - 25% | vorzugsweise | 9,1% |
|---|---|---|---|
| Polyvinylpyrrolidon | 15 - 25% | vorzugsweise | 22,7% |
| Mikrokörnige Cellulose | 30 - 50% | vorzugsweise | 43,2% |
| Maisstärke | 5 - 20% | vorzugsweise | 11,3% |
| Polyplasdone XL | 5 - 20% | vorzugsweise | 13,5% |
| Magnesiumstearat | 0,1 - 0,5% | vorzugsweise | 0,2% |

(die Prozentangaben sind Gew.%)

Nimodipin, mikrokörnige Cellulose und Polyplasdone XL werden mit einer acetonischen Polyvinylpyrrolidonlösung nach bekannten Verfahren der pharmazeutischen Technologie granuliert. Das Granulat wird mit Maisstärke und Magnesiumstearat vermischt.

Die so hergestellte Tabletten werden dann mit ca. 10 mg verschiedener filmbildenden Polymerisate nach den bekannten Methoden der pharmazeutischen Technologie beschichtet. Die Zusammensetzung der Filmen ist in Tabelle I wiedergegeben.

EP 0 548 595 A2

TABELLE I

| ZUSAMMENSETZUNG DER ÜBERZUGSFILME NIMODIPIN RETARD 45 mg | | ÜBERZUG | | | |
| --- | --- | --- | --- | --- | --- |
| | | "A" | "B" | "C1" | "C2" |
| POLYMER | HYDROXYPROPYLMETHYLCELLULOSE | 4,5% | | | |
| | HYDROXYPROPYLMETHYLCELLULOSENPHTHALAT | | 7,5% | | |
| | METHACRYLSÄURE COPOLYMERISAT, Typ A (USP/NF) | | | 15,0% | |
| | METHACRYLSÄURE COPOLYMERISAT, Typ B (USP/NF) | | | | 6,5% |
| PLASTIFIZIERMITTEL | PEG 4000 | 1,5% | | | |
| | TRIACETIN | | 1,2% | 2,25% | 1,0% |
| FARBSTOFFE / PIGMENTE | E 110 | | 0,005% | 0,0065% | |
| | $TiO_2$ | 1,0% | 1,0% | 1,0% | 1,0% |
| | $Fe_2O_3$ | | | | 0,2% |
| LÖSUNGSMITTEL | GEREINIGTES WASSER | 93,0% | 8,25% | 81,685% | |
| | ACETON | | 16,0% | | 20,0% |
| | ISOPROPYLALKOHOL | | 66,045% | | 71,3% |

Neben den Überzugstoffen, die in Tabelle I wiedergegeben sind, können auch andere Stoffe mit Verzögerungswirkung verwendet werden, z.B. Celluloseacetat-Phthalat, Hydroxypropylmethylcellulose-Phthalat 55, Polyvinylacetat-Phthalat.

Die Beständigkeit des durch Auftragung von Filmen aus organischem Lösemittel (siehe Tabelle I, Überzüge "B" e "C2") erhaltenen Überzuges wird beträchtlich durch eine weitere Hydroxypropylmethylcelluloseschicht gesteigert, die durch einen ca. 10 mg-Auftrag eines Filmes erzielt werden kann, der aus der in Tabelle I angeführten Zusammensetzung durch Zugabe von 0,005% E110 bzw. von 0,2% $Fe_2O_3$ erhältlich ist.

Hydroxypropylmethylcellulose kann durch andere Polymerisate ersetzt werden, z.B. Methylcellulose, Carboxymethylcellulose, Polyvinylalkohol, Polyvinylpyrrolidon.

So wird das pharmazeutische System der Erfindung erhalten bestehend aus einer Hartgelatinkapsel, die eine Kombination von drei oder mehr Tabletten mit unterschiedlich eingestellter Wirkstoffabgabe enthält; bei diesem System wird die erste Dosis therapeutisch wirksamer Substanz aus den Tabletten freigegeben, die in der Lage sind, die therapeutisch wirksame Substanz schnell dem Organismus abzugeben. Beide andere Dosen werden später durch Tabletten mit pH-abhängiger Auflösung freigesetzt. Die Anzahl der verschiedenen Tablettensorten kann je nach dem Gewicht zwischen einer und mehrere Einheiten variieren. Anstatt 164,8 mg-Tabletten kann man z.B. ca. 10 mg-Tabletten verwenden (mit einem Durchmesser von 2 mm). Auch solche Mikrotabletten werden mit verschiedenen Filmen mit Retard-Wirkung - nach dem in Tabelle I dargestellten Schema - überzogen. Die verschiedene Mikrotabletten werden dann vermischt und anschließend in 490 mg Dosen in Gelatinkapseln abgefüllt.

Dem Fachmann ist bekannt, daß die Entwicklung der pharmazeutischen Präparate mit anhaltender Wirkung immer eine in vitro Prüfung derer Löslichkeit vorsieht. Solche Tests wurden für die Mittel nach vorliegender Erfindung gemäß bereits experimentierten und nachfolgend angegebenen Bedingungen durchgeführt, um Löslichkeitswerte möglichst repräsentativ der biologischen Verfügbarkeit des Arzneimittels zu erhalten.

Da sich aus biologischen Untersuchungen der Endteil des Darmes ungeeignet für die Verwendung als Resorptionsstelle erwiesen hat, wurde für Nimodipin ein System gewählt, das die Verabreichung von zwei 45 mg- Dosen pro Tag vorsieht.

Zur Prüfung des pharmazeutischen Systems der vorliegenden Erfindung wurde die Wirkstoff-Freisetzung mittels einer Kapsel enthaltend drei Tabletten in 1,33 Liter von den in Tabellen III und IV angegebenen Lösungen festgestellt.

```
TABELLE III

PRÜFUNG DER LÖSLICHKEIT

NIMODIPIN RETARD: 45 mg

Menge (Abgabe %)

                              Zeit (Minuten)

. Stufe I    : 2 Std in 0,1 N HCl

+ Stufe II   : I + 2 Std bei pH 5,1

* Stufe III : II + 2 Std bei pH 6,5
```

TABELLE IV

PRÜFUNG DER LÖSLICHKEIT

NITRENDIPIN RETARD: 30 mg

Menge (Abgabe %)

Zeit (Minuten)

. Tablette A, in 0,1 N HCl

+ Tablette B, bei pH 5,2

* Tablette C, bei pH 7,5

Die Testkapsel wird in die drei Lösungen, deren pH-Werte in den Tabellen III und IV angegeben sind, gegeben und jeweils zwei Stunden darin stehengelassen.

Falls sich der magenresistente Überzug unerwünschterweise permeabel erweisen sollte, kann man etwa 2 mg eines hydrophoben Stoffes, wie z. B. Stearinsäure, Mischungen aus Triglyceriden der höheren Fettsäuren, hydrophobes Ricinusöl, Glycerylbehenat, und sämtliche in der pharmazeutischen Industrie gewöhnlich zur Bildung von wasserabstossenden Beschichtungen verwendete Stoffe als Unterüberzug zu dem genannten Film auftragen.

Der Auftrag eines wasserabstossenden Überzuges ist besonders für Stoffe wie Nimodipin notwendig, dessen Morphologie und folglich dessen Löslichkeit durch Wasser verändert werden kann.

Ein weiteres Beispiel einer Dihydropyridinverbindung, die mit dem pharmazeutischen System der vorliegenden Erfindung verabreicht werden kann, ist Nitrendipin.

**Beispiel II**

**Herstellung eines pharmazeutischen System zur Verabreichung von Nimodipin mit gesteuerter Freisetzung.**

Es werden 80 mg-Tabletten hergestellt, die 10 mg therapeutisch wirksame Substanz pro Tablette enthalten anhand folgender Einsatzstoffe:

| | | | |
|---|---|---|---|
| Nitrendipin | 5-15 mg | vorzugsweise | 10,0 mg |
| Maisstärke | 20-40 mg | vorzugsweise | 30,0 mg |
| Mikrokörnige Cellulose | 15-45 mg | vorzugsweise | 27,8 mg |
| Polyvinylpyrrolidon | 5-15 mg | vorzugsweise | 10,0 mg |
| Natriumlaurylsulfat | 1-3 mg | vorzugsweise | 2,0 mg |
| Magnesiumstearat | 0,1-0,4 mg | vorzugsweise | 0,2 mg |

Nitrendipin, 50% Maisstärke und mikrokörnige Cellulose werden mit einer wässrigen Polyvinylpyrrolidon- und Natriumlaurylsulfatlösung nach bekannten Verfahren der pharmazeutichen Technologie granuliert. Das Granulat wird mit Maisstärke und Magnesiumstearat vermischt und tablettiert. Die so hergestellte Tabletten werden dann mit ca. 10 mg Film beschichtet, um eine Kombination von A + B + C4/C3 Tabletten, deren Zusammensetzung in Tabelle II aufgeführt ist,

TABELLE II

| ZUSAMMENSETZUNG DER ÜBERZUGSFILME NITRENDIPIN RETARD 30 mg | | ÜBERZUG | | | |
|---|---|---|---|---|---|
| | | "A1" | "B1" | "C3" | "C4" |
| POLYMER | HYDROXYPROPYLMETHYLCELLULOSE | 4,5% | | | |
| | HYDROXYPROPYLMETHYLCELLULOSEPHTHALAT | | 7,5% | | |
| | METHACRYLSÄURE COPOLYMERISAT, A Typ (USP/NF) | | | 14,0% | |
| | METHACRYLSÄURE COPOLYMERISAT, B Typ (USP/NF) | | | | 7,5% |
| PLASTIFIZIERMITTEL | PEG 4000 | 1,5% | | | |
| | TRIACETIN RICINUSÖL | | 1,2% | 2,1% | 0,75% |
| FARBSTOFFE / PIGMENTE | $TiO_2$ | 1,0% | 1,0% | 1,0% | 1,0% |
| | $Fe_2O_3$ | | | 0,2% | 0,2% |
| | E 110 | | 0,01% | | |
| LÖSUNGSMITTEL | GEREINIGTES WASSER | 93,0% | 8,0% | 82,7% | |
| | ACETON | | 16,5% | | 20,0% |
| | ISOPROPYLALKOHOL | | 65,79% | | 70,55% |

in einer Gelatinkapsel, dessen Gesamtgehalt an Wirkstoff 30 mg beträgt, zu erhalten.

C4/C3 bedeutet, daß die Tablette mit Film C4 und danach mit Film C3 zur Steigerung des mechanischen Widerstandes - infolge eines Wirkungsmechanismus, der dem im vorstehenden Beispiel beschriebenen ähnlich ist - auf Grund des Auftrages eines Filmes aus wässrigem Medium über einen Film aus Lösemitteln, überzogen wurde. Aus demselben Grunde wurde die Tablette Typ "B" mit einem Film Typ "A" (zusätzlich enthaltend 0,01% E 110) überzogen.

Ein drittes Beispiel einer Dihydropyridinverbindung verabreichbar mittels des erfindungsgemässen pharmazeutischen Systems mit gesteuerter Freisetzung, ist Nisoldipin.

**Beispiel III**

**Herstellung eines pharmazeutischen Systems zur Verabreichung von Nisoldipin mit gesteuerter Freisetzung.**

Es werden 130 mg-Tabletten hergestellt, die 10 mg therapeutisch wirksame Substanz pro Tablette enthalten anhand folgender Einsatzstoffe

| Nisoldipin | 5-15 mg | vorzugsweise | 10,0 mg |
|---|---|---|---|
| Maisstärke | 20-40 mg | vorzugsweise | 30,0 mg |
| Laktose | 15-60 mg | vorzugsweise | 48,7 mg |
| Mikrokörnige Cellulose | 20-40 mg | vorzugsweise | 30,0 mg |
| Polyvinylpyrrolidon | 5-15 mg | vorzugsweise | 10,0 mg |
| Natriumlaurylsulfat | 0,5-3 mg | vorzugsweise | 1,0 mg |
| Magnesiumstearat | 0,1-1 mg | vorzugsweise | 0,3 mg |

Nisoldipin, Laktose mikrokörnige Cellulose und 50% Maisstärke werden mit einer wässrigen Polyvinylpyrrolidon- und Natriumlaurylsulfatlösung nach bekannten Verfahren der pharmazeutischen Technologie granuliert. Das Granulat wird mit der restlichen Mange Maisstärke und Magnesiumstearat vermischt.

Mit der obenangeführten Mischung werden 130 mg-Tabletten hergestellt, die - zur Erhaltung eines erfindungsgemäßen pharmazeutischen Systems - wie bei Nitrendipin beschrieben überzogen werden.

**Patentansprüche**

1.  Pharmazeutisches System zur Verabreichung von therapeutisch wirksamen Dihydropyridinen mit gesteuerter Freisetzung, dadurch gekennzeichnet, daß es aus einer Hartgelatinkapsel, die eine Kombination von wenigstens zwei verschiedenen Tabletten mit unterschiedlich eingestellter Wirkstoff-Freisetzung der genannten Dihydropyridinen enthält.

2.  Pharmazeutisches System nach Anspruch 1, dadurch gekennzeichnet, daß das therapeutisch wirksame Dihydropyridin Nimodipin ist.

3.  Pharmazeutisches System nach Anspruch 1, dadurch gekennzeichnet, daß das therapeutisch wirksame Dihydropyridin Nisoldipin ist.

4.  Pharmazeutisches System nach Anspruch 1, dadurch gekennzeichnet, daß das therapeutisch wirksame Dihydropyridin Nitrendipin ist.

5.  Verfahren zur Herstellung eines pharmazeutischen Systems zur Verabreichung von therapeutisch wirksamen Dihydropyridinen mit gesteuerter Freisetzung, dadurch gekennzeichnet, daß man nach an sich bekannten Methoden der pharmazeutischen Technologie Tabletten aus:

| Nimodipin | 5 - 25% |
| Polyvinylpyrrolidon | 15 - 25% |
| Mikrokörnige Cellulose | 30 - 50% |
| Maisstärke | 5 - 20% |
| Polyplasdone XL | 5 - 20% |
| Magnesiumstearat | 0,1 - 0,5% |

herstellt, die so erhaltene Tabletten anschließend nach an sich bekannten Methoden der pharmazeutischen Technologie mit je ungefähr 10 mg Film so überzieht, daß wenigstens drei verschiedene Tablettensorten erhalten werden, wovon die erste mit einem Film Typ "A" überzogen, die zweite mit einem Film Typ "B" überzogen und die dritte mit einem Film Typ "C1" bzw. einem Film Typ "C2" überzogen und gegebenenfalls die vierte mit einem Film Typ "C2" überzogen ist.

6. Verfahren zur Herstellung eines pharmazeutischen Systems zur Verabreichung von therapeutisch wirksamen Dihydropyridinen mit gesteuerter Freisetzung nach Anspruch 5, dadurch gekennzeichnet, daß zur Steigerung der Beständigkeit des Überzuges aus Filmen Typ "B" und Typ "C2" enthalten aus einem organischen Medium, diese Filme wiederum mit ungefähr 4 mg Hydroxypropylmethylcellulosefilm überzogen werden.

7. Verfahren zur Herstellung eines pharmazeutischen Systems zur Verabreichung von therapeutisch wirksamen Dihydropyridinen mit gesteuerter Freisetzung, dadurch gekennzeichnet, daß man nach an sich bekannten Methoden der pharmazeutischen Technologie Tabletten aus:

| Nitrendipin | 5-15 mg |
| Maisstärke | 20-40 mg |
| Mikrokörnige Cellulose | 15-45 mg |
| Polyvinylpyrrolidon | 5-15 mg |
| Natriumlaurylsulfat | 1-3 mg |
| Magnesiumstearat | 0,1-0,4 mg |

herstellt, die so erhaltene Tabletten anschließend nach an sich bekannten Methoden der pharmazeutischen Technologie mit je ungefähr 10 mg Film so überzieht, daß wenigstens drei verschiedene Tablettensorten erhalten werden, wovon die erste mit einem Film Typ "A1" überzogen, die zweite mit einem Film Typ "B1" überzogen, und die dritte mit einem Film Typ "C4" überzogen ist, letzterer wiederum überzogen mit einem Film Typ "C3".

8. Verfahren zur Herstellung eines pharmazeutischen Systems zur Verabreichung von therapeutisch wirksamen Dihydropyridinen mit gesteuerter Freisetzung nach Anspruch 7, dadurch gekennzeichnet, daß zur Steigerung der Beständigkeit des Überzuges aus aufgetragenen Filmen Typ "B1" enthalten aus einem organischem Milieu, diese Filme mit ungefähr 4 mg Film Typ "A1" beschichtet werden.

9. Verfahren zur Herstellung eines pharmazeutischen Systems zur Verabreichung von therapeutisch wirksamen Dihydropyridinen mit gesteuerter Freisetzung, dadurch gekennzeichnet, daß man nach an sich bekannten Methoden der pharmazeutischen Technologie Tabletten aus:

| Nisoldipin | 5-15 mg |
|---|---|
| Maisstärke | 20-40 mg |
| Laktose | 15-60 mg |
| Mikrokörnige Cellulose | 20-40 mg |
| Polyvinylpyrrolidon | 5-15 mg |
| Natriumlaurylsulfat | 0,5-3 mg |
| Magnesiumstearat | 0,1-1 mg |

herstellt, die so erhaltene Tabletten anschließend nach an sich bekannten Methoden der pharmazeutischen Technologie mit je ungefähr 10 mg Film so überzieht, daß wenigstens drei verschiedene Tablettensorten erhalten werden, wovon die erste mit einem Film Typ "A1" überzogen, die zweite mit einem Film Typ "B1" überzogen und die dritte mit einem Film Typ "C4" überzogen ist, letzterer überzogen mit einem Film Typ "C3".

10. Verfahren zur Herstellung eines pharmazeutischen Systems zur Verabreichung von therapeutisch wirksamen Dihydropyridinen mit gesteuerter Freisetzung nach Anspruch 9, dadurch gekennzeichnet, daß zur Steigerung der Beständigkeit des Überzuges aus aufgetragenen Filmen Typ "B1" enthalten aus einem organischem Milieu, diese Filme mit ungefähr 4 mg Film Typ "A1" überzogen werden.